# EUROPEAN PATENT SPECIFICATION

(11) **EP 1 907 052 B1**
(45) Date of publication and mention of the grant of the patent: **13.01.2010**
(21) Application number: 06751313.5
(22) Date of filing: 21.04.2006
(51) Int. Cl.: A61N 2/02

(54) **ELECTROMAGNETIC TREATMENT DEVICE**
ELEKTROMAGNETISCHE BEHANDLUNGSVORRICHTUNG
DISPOSITIF DE TRAITEMENT ELECTROMAGNETIQUE

(30) Priority: 21.04.2005 US 673398 P
(43) Date of publication of application: 09.04.2008
(73) Proprietor: KSM, Inc., Bellaire TX 77401-3311 (US)
(72) Inventor: HUNTER, Clifford, Wayne, Houston, Texas 77060 (US)
(74) Representative: Williams, Michael Ian
(86) International application number: PCT/US2006/015551
(87) International publication number: WO 2006/116354

(56) References cited:
- EP-A- 0 126 805
- GB-A- 2 217 990
- NL-C1- 1 001 559
- US-A- 4 066 065
- US-A- 4 993 413
- US-A1- 2002 103 411
- US-B1- 6 290 638

## Description

### CROSS-REFERENCE TO RELATED APPLICATIONS

This application claims priority to U.S. Provisional Application 60/673,398 filed on April 21, 2005 ("Provisional Application), which is incorporated herein by reference along with its appendices.

### FIELD OF THE INVENTION

The present invention relates to the field of medicine and alternative medicine. More particularly, the invention relates to a system for treating subjects with a magnetic field.

### BACKGROUND

The human body produces subtle electromagnetic fields, which have been generated in the body through chemical reaction within cells and ionic currents passing through the nervous system. In recent years scientists have been discovering ways that electromagnetic fields influence the body's functioning both in a positive as well as a negative manner. These observations and others have led to the development of electromagnetic therapy as an alternative medicine.

Alternative medicines and therapeutic treatments have been developed to treat disease, illness, and injury. Alternative treatments comprise herbal potions, therapeutic massage, acupuncture, chiropractic manipulation, yoga, and other techniques. Diet and nutrition techniques have supplemented medicinal remedies for disease such as diabetes. Numerous techniques have been developed to treat disease, illness and injury with different forms of electrical energy. Representative examples include United States Patent No. 3,670,737 which disclosed a device for administering high frequency oscillations to a patient. The treatment duration was controlled to limit undesirable heat generation. United States Patent No. 4,621,642 disclosed a microwave apparatus for contacting a patient with a multi-frequency output. United States Patent No. 5,186,171 disclosed a high voltage, high frequency pulsed source for treating musculoskeletal disorders. Numerous other techniques have been developed for the application of different energy forms to a body, however the relative success of such techniques is inconsistent.
Document US 6641520 discloses a magneto therapy system according to the preamble of claim 1.

Although existing techniques are available to treat certain illness and disease, improvements in, additions to and complements of such treatments would enhance the quality of life and ameliorate or reduce symptoms associated with a variety of conditions. A need exists for additional systems and methods capable of complementing treatment of certain disorders, abnormalities, and diseases. The present invention provides a system for exposing all or part of a subject to a magnetic field.

### SUMMARY OF INVENTION

The object of this invention is solved by a system according to claim 1. Various embodiments are disclosed in the dependent claims.

Embodiments of the system comprise a direct current electricity source, a coil that is activated by the source to generate a magnetic field, and an aperture within the coil for permitting insertion therein of one or more appendage or part(s) of a subject so that components of the blood or other tissues are subjected to the magnetic field.

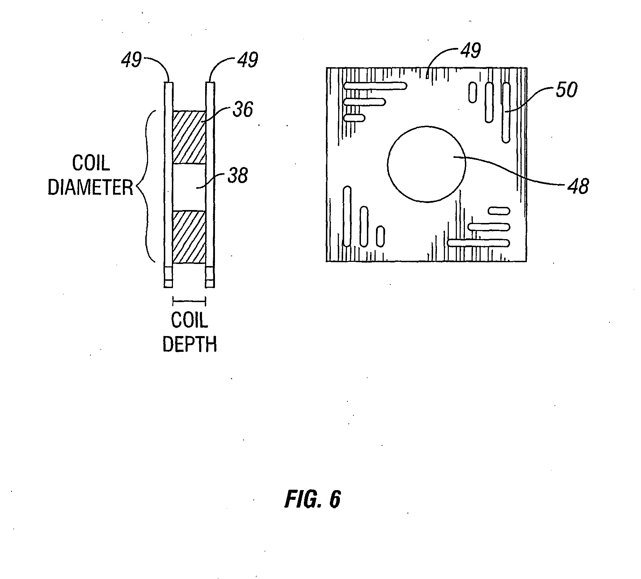

Certain embodiments of the invention include a system for treating subjects and the components of their blood or tissues, including but not limited to blood borne cells, proteins, solutes and other components. These components may be circulated via the blood or lymph within a subject. A system of the invention typically comprises: a) a direct current electricity source; b) a coil that can be activated by the source to generate a magnetic field; and c) an aperture within said coil for permitting insertion therein of an appendage for the person so that blood cells within the appendage are subjected to said magnetic field. In certain aspects, the system is capable of modifying blood cells within the appendage for treatment of a subject at a location exterior to or distal to the appendage. In various embodiments the aperture is capable of permitting insertion of the person's hand, foot, wrist, ankle, lower leg, upper arm or any other body part, including the whole body of smaller animals. Preferably, the diameter of the coil is greater than the depth of the coil, more preferably the diameter may be 2 to 20 times that of the depth of the coil. Diameter is ' determined by the length across the vertical plane forming a circular cross-section (circular plane) of the coil, see Figure 6. Depth is the length measurement along the perpendicular from the circular plane of the coil, see Figure 6. The system may further comprise a heat exchanger for removing heat from said coil.

The system may further comprise a housing around the coil, a fluid within the housing, and a conduit for conveying the fluid from the housing to the heat exchanger. In a preferred embodiment the housing is manufactured from a metal such as steel. The system may further comprise a pump for circulating the fluid through the housing and the heat exchanger. Preferably, the fluid is a non-conducting fluid with the appropriate properties for heat exchange, such as silicon oil, and more preferably silicon transformer oil.

In yet another embodiment, the system may include various sensors. Such sensors typically include a sensor for monitoring the temperature of the coil, a sensor for monitoring the voltage and current passing to the coil, or both, a sensor for monitoring the temperature of the coil and a sensor for monitoring the voltage and current passing to the coil. In another aspect, the coil is capable of modifying the relative orientation of the blood cells, modifying the magnetic properties of the blood cells, or both modifying the relative orientation of the blood cells and modifying the magnetic properties of the blood cells.

In still yet another embodiment, a system of the invention may be used for treating a subject. The methods include using a system comprising: a) a housing for a direct current electricity source; b) a coil that is activated by the source and generates a magnetic field; c) an aperture within the coil for permitting insertion therein of an appendage for the subject so that blood within the appendage are subjected to the magnetic field; and d) a heat exchanger engaged with the coil for removing heat from the coil. In a preferred embodiment, the aperture is lined with a stainless steel cylinder or sleeve. In still another preferred embodiment the housing is manufactured from a metal or steel. Typically, this sleeve will be continuous with the housing forming the enclosed module casing. An insulating sleeve with an interior diameter larger than the exterior diameter of the stainless steel sleeve, which is constructed of a non-conducting material, such as plastic or ceramic material, is positioned around the stainless steel sleeve. This insulating sleeve is inside the module. According to the invention, there is a gap between the insulating sleeve and the inner sleeve through which cooling fluid inside the module can flow. This fluid flows between the sleeves, cooling the inner stainless steel sleeve.

The system may be used to reduce or ameliorate various symptoms related to **a** variety of conditions such as, but not limited to pain, increase blood cell circulation, and decrease cell adherence. Generally, the treatment may improve the subject's symptoms related to sickle cell anemia, diabetes, abnormal blood pressure, skin cancer and prostate and other cancers, arterial sclerosis, strokes, heart angina, AIDS related illnesses and complexes, or fibromyalgia. The system may also be used to ameliorate or reduce the symptoms of epilepsy, Alzheimer's Disease, Parkinson's Disease, arterial sclerosis, strokes, heart angina, leukemia, Lou Gehrig's Disease, cerebral palsy, sickle cell anemia, or multiple sclerosis.

Furthermore, the system may be used in ameliorating, reducing or improving the one or more symptoms of lupus, arthritis, fibroid tumors, neuropathy, sinus abnormalities, sprains, trauma, vision deficiencies, or migraine headaches.

### BRIEF DESCRIPTION OF THE DRAWINGS

**Figure 1** illustrates a schematic diagram for a system having four treatment modules.

**Figure 2** illustrates an elevation view of a coil and interior port.

**Figure 3** illustrates a schematic diagram for an electrical circuit activating four modules.

**Figure 4** illustrates a schematic of an exemplary cooling system for an ETD.

**Figure 5** illustrates a schematic of an exemplary electrical connector.

**Figure 6** illustrates an example of a coil spindle plate.

**Figure 7** illustrates an example of a system used for the treatment of a person.

### DESCRIPTION OF THE PREFERRED EMBODIMENTS

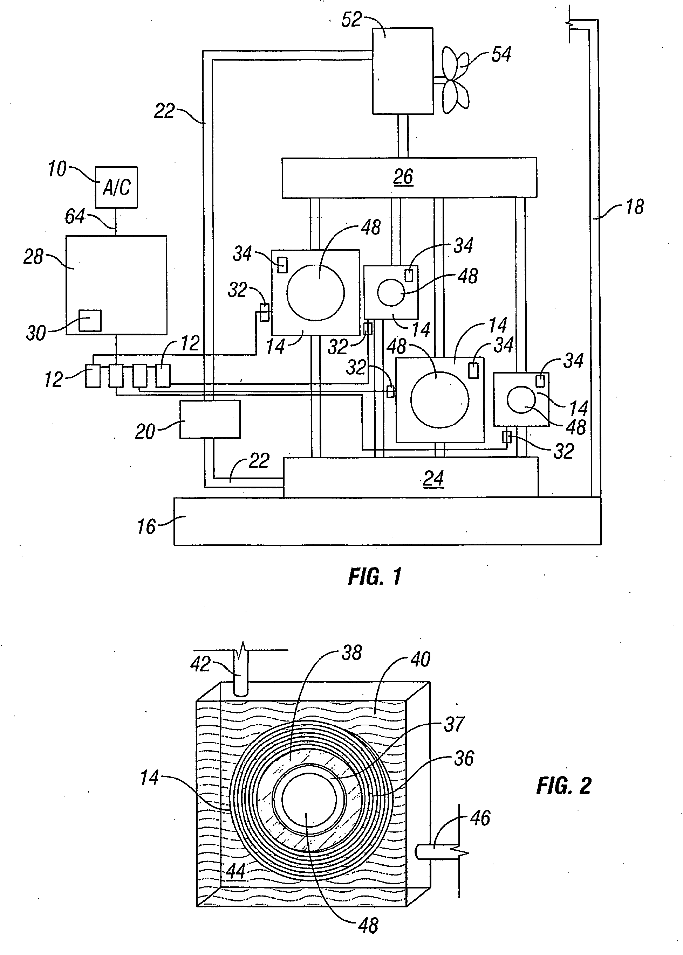

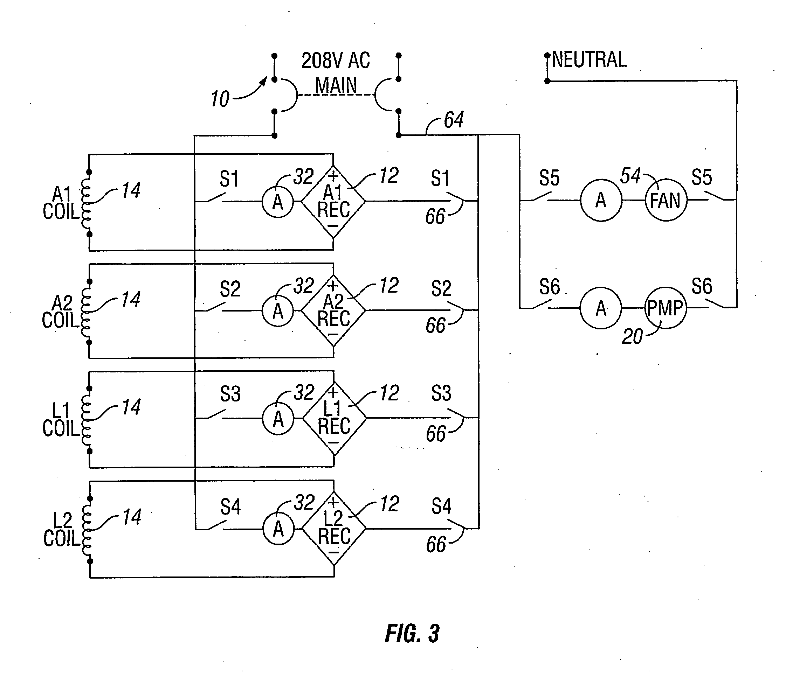

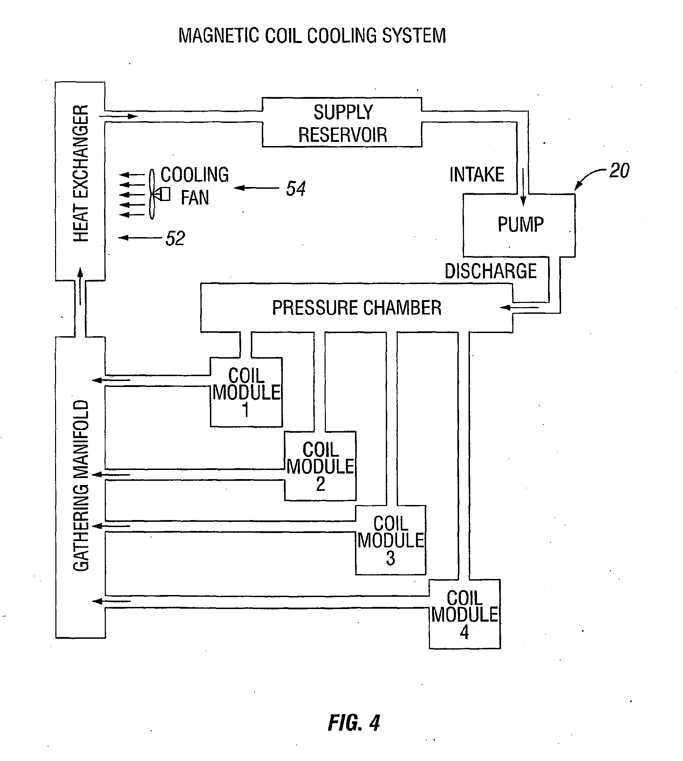

Typically, methods of the invention permit blood passing through an arm or foot to pass through a homogeneous magnetic field created by an electromagnetic coil. Cells within the blood circulated in the appendage are passed through the homogeneous magnetic field as the appendage is maintained within the magnetic field. A schematic for such system is illustrated in Figure 1 wherein alternating current source 10 is connected to rectifiers 12 to convert the alternating current to direct current. Modules 14 identified as magnetic coils are connected to frame 16 and covered by cover 18 which can be formed with a material such as stee. Alternatively, the cover 18 may be 304 grade polished stainless steel. The system incorporates one or more coil modules 14 illustrated in Figure 2, electric circuitry illustrated in Figure 3, cooling elements such as pump 20, conduit 22, pressure chamber 24, and gathering manifold 26, illustrated in Figure 4. Control panel 28 provides manual or automatic capabilities to monitor and adjust the system operation, and can be operated with microprocessor 30. Current gauges 32 monitor electric flow to each magnetic coil 14, and proper operation can be verified by monitoring the current flow relative to the measured voltage. Sensors 34 monitor the temperature of coils 14 and are connected with control panel 28 to avoid overheating of coils 14.

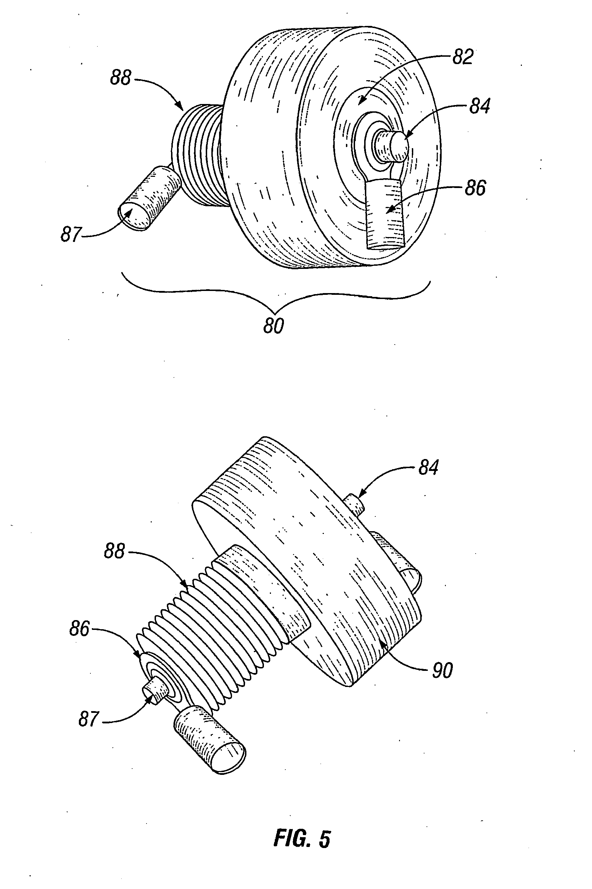

Magnetic coils 14 are formed with wire coil 36, insulating sleeve 38, and housing 40 as illustrated in Figure 2. Inlet 42 permits entry of fluid 44 into contact with wire coil 36, and outlet 46 permits exit of such fluid 44. Figure 5 illustrates an exemplary electrical connector 80 for the housing that reduces fluid leakage from the housing via the electrical connection. Electrical connector 80 comprises a conductor post 84 that provides an external terminal connection 86 and an internal terminal connection 87 that span the housing 40 and connect the coil 36 to the power source (rectifiers) 12. The connector 80 may be attached by threaded housing spanning member 88. The connector may be attached using various washer and gasket configurations, which include a gasket and/or washer positioned between the external connector body 90 and the housing 40.

Insulating (second) sleeve 38 is preferably formed with a nonmagnetic material such as CPVC plastic to protect a subject's appendage from direct electrical contact with wire coils 36. According to the invention, there is an inner sleeve 37 inside the insulating sleeve 38. A gap is provided between insulating sleeve 38 and inner sleeve 37 to allow fluid 44 to flow between. The inner sleeve is preferably a stainless steel sleeve. Each magnetic coil 14 has an interior aperture or port 48 for permitting insertion of a subject's body appendage. Such ports 48 can be different sizes depending on the size and character of the subject's appendage undergoing treatment. In certain embodiments, the coil depth may be approximately 10, 20, 25, or 30% of the coil diameter. Valves in this range cause the magnetic field to be more homogenous than with large valves. Homogeneity of the magnetic field will typically cause saturation of mass in less time.

Figures 1 and 4 illustrate exemplary cooling systems, however other configurations would be operational with the invention. The resistance within the module coils generates significant heat which would be undesirably transferred to the subject. The system removes such heat by pumping oil, such as silicon transformer oil, through the closed jacket or housing surrounding each coil 36. Oil is circulated from jacket to heat exchanger 52 having cooling fan 54. In still further embodiments there are two reservoirs of coolant, one reservoir may be a pressure reservoir and the second may be a vacuum reservoir. Typically, the coolant is pumped through the modules to the heat exchanger through one or more reservoirs.

Each treatment module has an inlet and outlet for circulating oil through the module. A single pump 20 may supply oil under pressure and return such oil to a supply reservoir, which may be in communication with the suction side of pump 20. Control panel 28 is engaged with each coil 14 to monitor continuously for voltage and amperage. Deviations from normal operating ranges will signal a warning and cease operation of the alarm module. A temperature control system is independently operated by a temperature switch 34 engaged with each coil 14, and all operating controls can be locked within access panel 28.

Coil modules are typically cooled with an oil bath circulated through the housing. In certain configurations, coil spindle plates 49 are perforated by holes or slots 50 in a variety of patterns to allow passage of coolant to cool the modules (Figure 6). The slots or holes in the spindle plates 49 may take 20 to 90 % of the area of the spindle plate 49 and may be positioned to completely, and is certain embodiments uniformly, cover the area of the plate. Figure 6 also illustrates the diameter and depth of the coil. Oil is directly contacted with magnet wire by pumping oil through multiple perforations in the spindle walls supporting the coil. Each module has an inlet and outlet for circulating the oil. In a preferred embodiment of the invention, silicon type transformer has been discovered to provide superior cooling.

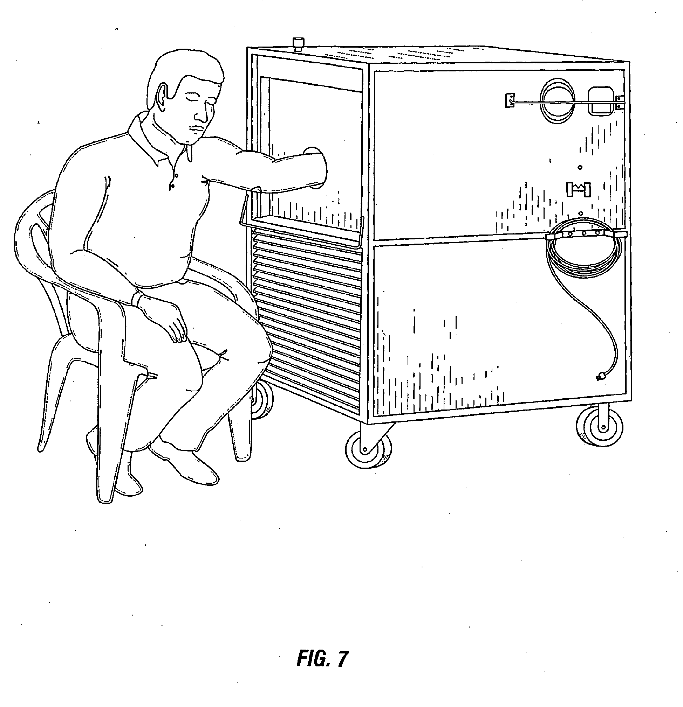

In certain embodiments, the module housing is made from ferrous materials to absorb magnetic fields, preferably steel. Typically, the modules will be constructed using plastic or ceramic material as a core (insulating sleeve) to wrap the wire, whereas the casing will typically be made of metal. In other embodiments, the cover 18 and external housing or cabinet shown in FIGURE 7 is also made from ferrous materials. The use of ferrous materials has been shown to reduce the extraneous magnetic field by up to 75%, allowing patients with pacemakers to use the system. In still further embodiments, the port, aperture or opening corresponding to a port will be comprised of the inner diameter of an inner sleeve. Preferably, the inner sleeve is made of stainless steel, which may cause the magnetic field to be focused in the opening and within the module.

In a preferred embodiment, the typical coil specifications are as follows: a) a 10.2cm (4-inch) port will typically include 36.7kg (81 pounds) of #18 magnet wire (4970m (16,300 feet) of wire), resulting in 104 ohms resistance, with about 6800 turns. Inside dimensions of the coil are 10.8cm (4.25 inches) in diameter with outside dimensions 35.6cm (14.0 inches) in diameter; b) a 15.2cm (6-inch) port will typically include 36.3kg (80 pounds) of #18 magnet wire (4901m (16,100 feet) of wire), resulting in 103 ohms resistance, with about 5,800 turns. The inside dimensions of the coil are 15.9cm (6.25 inches) in diameter with an outside dimension of 38.1cm (15.0 inches) diameter; c) a 20.3cm (8-inch) port will typically include 37.6kg (83 pounds) of #18 magnet wire (5090m (16,700 feet) of wire), resulting in 105 ohms resistance, 5050 turns. An inside dimension of the coil is 21m (8.25 inches) in diameter and an outside dimension is 43.7-cm (17.0 inches) in diameter.

In still other embodiments, a treatment port is 10.2cm (4.0 inches) in diameter and is insulated by a CPVC insulating sleeve 0.32cm (0.125 inches) thick, with a 10.8cm (4.25 inch) outer diameter. In one embodiment, the coil can comprise 37.6kg (83 pounds) of No. 18 magnet wire having an outside diameter of 28cm (11 inches), with a thickness of 6.4cm (2.5 inches), and having 10,1,00 turns. Field strength at the design voltages are: a) 110 volts - 1.1 amperes current with 10,100 turns and coil strength of 11,100 ampere-turns; b) 220 volts - 2.2 amperes current, 10,100 turns and a coil strength of 22,200 ampere-turns; or c) 440 volts - 4.4 amperes current, 10,100 turns and a coil strength of 44,400 ampere-turns.

In another embodiment of the invention, the treatment port is 20.3cm (8.0 inches) in diameter and the outside of the sleeve is 21cm (8.25 inches). Coil specifications are 36.3kg (80 pounds) of No. 18 magnet wire, the outside diameter of the wire is 31.1cm (12.25 inches), and there are 6,000 turns. Field strengths at the design voltages are: a) 110 volts - 1.1 amperes current with 6,000 turns and coil strength of 6,600 ampere-turns; b) 220 volts - 2.2 amperes current with 6,000 turns and a coil strength of 13,200 ampere-turns; or c) 440 volts - 4.4 amperes current with 6.000 turns - and a coil strength of 26,400 ampere-turns.

In another embodiment of the invention, the treatment port is 15.2cm (6.0 inches) in diameter and the outside of the sleeve is 15.9cm (6.25 inches). Coil specifications are 36.3kg (80 pounds) of No. 18 magnet wire, the outside diameter of the wire is 27.3cm (10.75 inches), and there are 9,400 turns. Field strengths at the design voltages are: a) 110 volts - 1.1 amperes current with 9,400 turns and coil strength of 10,300 ampere-turns; b) 220 volts - 2.2 amperes current with 9,400 turns and a coil strength of 20,700 ampere- turns; or c) 440 volts - 4.4 amperes current with 9,400 turns and a coil strength of 41,300 ampere-turns.

Figure 3 illustrates one embodiment of a control panel 28 and associated system wiring. Normal line power voltage is 220 volts, although the system is capable of operating at different voltages as illustrated above. Line voltage is delivered to the device by conductor 64 connected to control panel 28. Line voltage is then delivered to a double-pole, single throw 40 amp 600 volt-rated switch 66. When each switch 66 is turned on, switch 66 feeds a 25 amp rated full-wave bridge rectifier 12 associated with each coil 14. One leg of the AC form switch is routed to a 20 amp rated ammeter 32 to measure current used by each coil 14. Design of the device produces currents use of 2.2 amps at 220 volts, 1.1 amps at 110 volts, and 4.4 volts at 440 volts. From each rectifier 12, a positive and negative DC voltage is fed to the coil 14 being served, and an electromagnetic field is generated. Each treatment is served by a separate ammeter 32 and rectifier 12.

Operation of the system permits insertion of a patient's appendage such as a hand, arm, foot, or leg into one of the ports associated with a coil 14. Such placement into the module port positions the appendage so that the appendage is exposed to the coil 14 magnetic field at the most effective location. The configuration and operation of coil 14 generates a substantially homogeneous magnetic field for treatment of the body appendage. Treatment is systemic and treats the entire theoretical volume of a patient's blood, therefore providing effective treatment without requiring direct contact between the system and the diseased or injured body part.

Although the invention has been described in terms of certain preferred embodiments, it will become apparent to those of ordinary skill in the art that modifications and improvements can be made to the inventive concepts herein without departing from the scope of the invention. The embodiments shown herein are merely illustrative of the inventive concepts and should not be interpreted as limiting the scope of the invention.

The present invention provides a system for treating symptoms related various diseases or injured states of a subject by treating the biological components circulating throughout the body. A blood cell theoretically circulates through a person's body once every fifteen minutes. Within this time interval, a single blood cell beginning at the wrist or foot would theoretically travel completely through the body before returning to the initial point. With such fifteen minute circulation cycle, a thirty minute time interval would theoretically define two complete blood circulations.

Blood cells nourish all body components including soft tissue, organs, bone, and the nervous system. Abnormal blood cells may bind one cell to another and form "lumps" of cells within the circulatory system. Symptoms of abnormal blood cells sometimes include overall fatigue, breathlessness, and lethargy. Abnormal blood cells further reduce the body capacity to improve or recover from disease or abnormal conditions.

The present invention implements a magnetic field, preferably a homogenous magnetic field, for treatment of individual blood components, preferably within a subject's appendage, such as an arm, hand or foot to provide benefit to other bodily components.

As previously noted, agglomeration of blood cells generates or exacerbates abnormal conditions within a body. The invention permits a body appendage and internal blood cells to be exposed to the energy from the magnetic field created by exciting a coil with direct current electricity. The coil design provides magnetic field strength at approximately the same level at any point in a treatment opening within the coil. After application of the invention, microscopic evaluation of the blood cells typically reveals that the cell agglomerations are disaggregated into individual cells.

Successful treatment of various conditions by the invention has been clinically witnessed, including successful treatments for ameliorating the symptoms of diabetes, abnormal blood pressure, skin cancer, and prostate cancer, as well as other cancers, multiple sclerosis, lupus, arthritis, epilepsy, Alzheimer's Disease, Parkinson's Disease, arterial sclerosis, strokes, angina, leukemia, Lou Gehrig's Disease, fibroid tumors, neuropathy, sinus abnormalities, sprains, trauma, vision deficiencies, migraine headaches, AIDS related illnesses and complexes, sickle cell anemia, fibromyalgia, cerebral palsy, and other symptoms. Various forms of circulation have been improved, including blood and lymph circulation.

In a group case study regarding chronic pain syndrome clinically diagnosed as predominantly comprising fibromyalgia, a group of seventy patients represented predominantly intractable pain sufferers with symptom durations ranging from six months to approximately forty years, and were treated with a total of two hundred treatments (see Provisional Application, incorporated herein by reference in its entirety). Most were diagnosed as suffering from fibromyalgia, a disease without an established effective treatment. In prior blinded studies of this disorder virtually no demonstration of placebo effect had been observed.

Experimental treatment by the methods and devices of the invention consisted of hand or foot insertion into the system portholes described below. The experiments used three different machines with a total of ten different magnetic ports of varying field strengths. Treatment times varied from sixteen minutes to two hours. Certain subjects were switched from one port to another within the course of a single treatment. A substantial number of subjects reported different sensations during treatment which were described as neutral and not unpleasant.

More than half of the subjects reported significant degrees of pain relief. Another quarter of the subjects reported degrees of pain relief sufficient to decrease analgesic requirements, but not adding functional capacity. Within the group experiencing significant functional improvement, some experienced improvement in related symptoms such as better cognition, clearing vision, reduced sicca symptoms, warming of hands and feet, improvement in paresthesias, and improved bowel and bladder function. Benefits from a single treatment generally lasted between two and seven days, and it is believed that additional repeat treatments may provide serial effect.

Of the seventy subjects within the study group, fewer than twenty reported no effect. In addition to the positive benefits and neutral reactions were generally described. In fact, one subject experienced significant improvement. This subject had experienced paralysis of the left hand for over thirty-five years caused by an intracerebral hemorrhage. The subjects hand was tightly flexion-contractured. Following treatment the contractured hand slowly opened and became usable. Near normal grip strength was apparent following a two hour treatment. After some function was restored, the subject was able to use the left hand for normal activities. Such use resulted in a stress fracture of the middle phalanx of the fifth digit superimposed on a background of profound Sudek's atrophy, which was not caused by the treatment itself, but is notable because such fracture could not have occurred absent the functional improvement caused by the treatment.

Positive results for the diseases, conditions, and abnormalities listed herein have been obtained without any detectable adverse side effects. Additionally, positive changes in blood elements have been detected, representing measurable benefits in treating one or more of the ailments. Typically, alcohol consumption negatively effects the positive benefits of the treatment.

### THE ELECTROMAGNETIC TREATMENTDEVICE (ETD)

The Electromagnetic Treatment Device (ETD) typically contains four electromagnetic coils each with an opening (or port) at its center that allows a body part or appendage, i.e., a hand, arm, foot or leg, to be suitably positioned to efficiently be exposed to a continuous DC magnetic field of the order of 0.5 Tesla.

### EXEMPLARY FEATURES OF A SYSTEM

Features of a typical ETD include, but are not limited to safety locked controls; an integrated cooling system; the use of static magnetic fields, which produce minimal extraneous magnetic fields; non-invasive patient treatment; simple non-adjustable on-off controls; operation status metering for each module; operation off standard 220 volt wall power, electrical safety tested, and wheel mounted. Provisional Application, incorporated by reference in its entirety, provides additional examples in the form of photographs and schematics related to the present invention.

### SYSTEM DESCRIPTION

In certain embodiments of the invention described generally above and the figures, a device may be approximately 2m x 0.7m x 1.2m (6 ½' x 2 ½' x 4') high and weighing in excess of 180kg (400 pounds). The device may contain four solenoid magnetic field treatment coils with central openings of 10.8cm, 10.8cm, 15.9cm, and 21cm (4.25", 4.25", 6.25", and 8.25") diameters and in certain embodiments approximately 10.2cm (4") in length. The unit is powered by single phase, 220 volt AC. The AC is rectified to approximately 220 volt DC by separate full-wave bridge rectifier circuitry to each coil. AC power is supplied to a pump and fan providing circulation of dielectric oil to each coil. The unit is connected by a 220- volt, 20 ampere, three wire, grounded, UL approved power cable. In operation each coil draws approximately 2.3 amperes and the unit with all systems operating draws approximately 17 amperes. AC power switches are separately provided for main power, each coil, pump and fan. The coils may be surrounded, with the exception of the aperture, by a closed steel housing, alternatively stainless steel, that may be filled with silicon dielectric oil for additional insulation and cooling. All coil, pump, fan, and power circuitry housings are solidly electrically grounded to the metal structural frame. The frame is electrically connected to the ground wire of the power cable. All operating features are typically completely and only controlled by on-off switches. Computer microprocessor or software systems need not be employed. The main electrical function is to provide DC Current to the magnetic coils. All circuitry is of standard design and implemented with proven robust conservatively rated components. A schematic of an ETD is provide in the Figure 7. The front side as shown is the preferred location of the controls and electrical measurement meters. It may have a lockable front cover. The arm/wrist treatment ports may be located on one side of the unit. The leg/ankle ports may be on a different side of the machine, preferably opposite the arm/wrist ports. Typically, the leg/ankle ports will be located and lower on the unit for accommodating access.

### CONTROL AND METERS

### Physical Access

All controls are located behind a hinged lockable front panel. During operation it is contemplated that the panel remain unlocked for quick access to all controls.

### Controls

The system controls are typically simple On-Off switches, however the inventor contemplates that variable types of switches may be used. There is no apparent need for variable or adjustable controls. There may be various types switches including one or more switches separately controlling the power to a treatment coil, each switch should be clearly labeled to indicate the treatment module it controls; at least one second switch controls the coolant circulation pump, and a third switch may control the cooling fan for the heat exchanger. It is advisable that when a treatment module is on both the fan and pump are also on. The fan and pump may be operated individually without damage provided no module is powered.

### Metering Devices

Adjacent to each switch is an ammeter that indicates the current being drawn to the load controlled by that switch. Each ammeter is also separately labeled with the same number as to its related switch and load. The meters are physically located so that they cannot be easily confused. The meters for the switches to the left are on the left of the corresponding switches and the meters for the switches to the right are on the right of the corresponding switches. The meters are all identical to avoid confusion. They read to a maximum of 10 amperes of AC current with subdivisions of 2 amperes. The four meters related to power to the magnetic coils measure the AC currents being supplied before rectification. The pump and fan ammeters measure the AC current drawn by the respective units. A temperature monitoring device, such as a thermometer, may also be installed in or around a treatment port or coil to measure the temperature at each treatment module.

### ELECTRICAL

The unit may be powered by 220 volt AC, 20 amperes, 60 Hz; Rectified to DC. Main power switches may include, but are not limited to individual on-off switch for each operating module, the pump and the fan. There may also be provided a locked key access to control panel or some other mechanism to regulate access to the controls.

The unit may further comprise dielectric isolation including insulation with wide safety margins, particularly between active coils and patient. The insulation may include wiring dielectric interfaces and silicon dielectric insulation oil. The oil may also serve as a coolant. A patient's arm or leg may rest within a dielectric cuff.

Typically, AC current will be rectified to DC current. The AC power is typically connected to full-wave bridge rectifier circuit(s) that provide the DC voltage to the treatment coil(s) through control switches. An exemplary electrical schematic is illustrated in Figure 3.

### MAGNETIC FIELDS

### Treatment Fields

The ETD exposes a portion of the arm and hand or the leg and foot to a DC magnetic field. For safety considerations the magnitude of this field can be compared to the magnetic fields approved for use in Magnetic Resonance Imaging (MRI) and Magnetic Resonance Spectroscopy (MRS) diagnostic devices. The magnetic fields in such devices can expose the whole patient body to levels of 1.5 to 2 Tesla (15,000 to 24,000 Gauss). The ETD exposes only extremities of the patient to levels approximately 3% to 5% (300 to 700 gauss) of a typical MRI device. The coil being a solenoid produces a magnetic field, which rapidly decreases in magnitude within short distances from the end of the coil. The field is less than 20 gauss at 12 inches from the coil. The range of values for the magnetic field across the treatment area, approximately the coil length plus 5.1cm (2") on each end, are given in Table 1. The high value is the magnitude of the field at the middle of the coil on the centerline of the coil.

**Table 1. Exemplary values for magnetic field.**

| **MODULE** | **EXTREMITY EXPOSED** | **COIL APERTURE** | **WORKING FIELD MAGNITUDE** |
|---|---|---|---|
| 1 and 2 | Arm and Hand | 10.8cm (4.25") | 150-500 Gauss |
| 3 | Leg and Foot | 21cm (8.25") | 100-300 Gauss |
| 4 | Leg and Foot | 15.9cm (6.25") | 125-400 Gauus |

The magnitude of the magnetic field can not increase. The field is at maximum when device is on. Sudden cessation of magnetic field is typically not detrimental to patient or treatment.

### Extraneous Magnetic Fields

The magnetic field coils are of solenoid design. This closed form arrangement gives a fast fall-off of magnetic field magnitude away from the open apertures of the coil. Within 0.3m (one foot) from the coil the magnitude of the field is reduced by more than one order of magnitude with a value of less than 5% of the maximum field in the treatment volume. At 1.5m (five feet) from the machine, the magnitude of the magnetic field is at a level commonly found around many types of standard operating electrical equipment or power source electrical utility service and less than five times the earth's natural magnetic field. Measured magnetic field values at several locations from the treatment volume are given in Table 2.

**Table 2. Extraneous Magnetic Field Levels.**

| **30-5cm (12"), Hemisphere Centred at Coil Aperture** | **61cm (24") Hemisphere Centred at Coil Aperture** | **1.5m (5 Feet) from Unit** |
|---|---|---|
| <20 Gauss | <5 Gauss | <1 Gauss |

### MECHANICAL

### Pump

An example of a coolant circulation pump is a Model TE-6-MD- SC-c-csa 586504 (Little Giant Pump Company). The Pump is powered by a ½ hp motor with electrical requirements of 230 volts, 60 Hz, 4.5 amps. All electrical power is provided with bard wire direct connections internal to the ETD. The power to the pump may be controlled by a control switch on the system control panel as previously described.

### Fan

An exemplary cooling fan is manufactured by Thermal Transfer Products LTD. The power to the fan may be controlled by control switch on the system control panel as previously described.

### Heat Exchanger

An exemplary heat exchanger is manufactured by Thermal Transfer Products LTD. Typical elements and coolant paths are illustrated by the flow chart in Figure 5.

### OPERATION OF THE SYSTEM

Preferably, a unit shall be installed in a dedicated room with controlled access so that objects and personnel will not be affected or harmed by the extraneous magnetic field that is present when any of the modules are operating. The unit should be position so that the power cable is in a position out of any walk path by patient or operator. The unit power cable may be connected to a standard GFI wall outlet rated at 220-volt and 20 amperes. It is advisable that the outlet be feed from a 220-volt, single phase, minimum 20-ampere source and fused or over current breaker rated for 20 amperes. However, one familiar with electrical device would be able to modify such a device to integrate with other power sources. The room air conditioning system should be capable of maintaining ambient temperatures in the range of 21°C (70°F) to 25.5°C (78°F) for a preferred operation temperature of the unit and for patient comfort.

### OPERATION

It is advisable not to plug the unit into the power outlet until all preliminary safety precautions are complied with. Check as a minimum that a) the room has been examined for any loose ferromagnetic objects and all such objects removed and similar objects on any personnel should also be removed such as jewelry, knives, watches, etc.; b) access to the room is limited to the persons performing the system operation and those required to perform the methods; and c) all switches on the unit are in the "off position.

After the initialization and/or safety procedures have been carried out the unit power cable connector may be plugged into an appropriate electrical outlet. Next, turn on both the pump and fan power switches. Turn on each module separately and check for proper current draw. Turn on all modules. Monitor temperature gauges and check that the temperature does not rise too more than 10% above ambient.

The cooling system should be engaged prior to the unit being operated for any substantial length time. The cooling system is designed to provide for removal of heat generated by resistance heating within the wires of the magnetic coils. The coils generate about 450 watts of thermal power. With all modules operating approximately 2 kilowatts of thermal power is transferred to the ambient air by the cooling system. The heat exchanger is operated by forced ambient airflow by the fan. The coolant fluid and hence the coils are maintained at a temperature about ten degrees above ambient room air temperature.

It is advisable that the cooling system be operating when the magnetic field modules are energized. While the coils may be cooled for a period of time by the presence of the non-flowing coolant they will eventually heat to a temperature that will permanently damage the coils and their insulation resulting in electrical arcing and possibly cause a fire within the device. If overheating occurs resulting in system temperatures in excess of 49°C (120°F) the unit should not be operated and appropriate repair sought.

### MAINTENANCE

The electrical and mechanical components have been chosen and used in a conservative rated manner. However, as with all electrical powered and mechanical motion equipment, component degradation or failure is possible. Routine observation and recording of operational parameters is important to safety and equipment life. Other than observation and recording of the parameters noted, as well as sanitation of the case and module(s), the device requires minimal maintenance. It is advisable that only factory authorized and trained personnel should repair the device or its components. After any electrical repairs the unit needs to be retested to assure meeting required safety standards.

The general specifications for some components of the device preferably include, but are not limited to a) input power - 220 volt AC, single phase, 20-amp, 60 Hz, b) magnetic coils producing magnetic fields of 100 - 500 gauss, c) coolant - silicon transformer oil, and d) pump providing maximum flow rate of 38 GPM.

### SAFETY MEASUREMENTS

Exemplary safety measure include electrical safety tests using a QuadTech, Model Guardian 6100, Medical Production Safety Analyzer with current calibration certification. Safety test measurements that may be made include to (1) Ground Continuity, (2) Ground Bonding, (3) AC Hipot, (4) DC Hipot, (5) AC Insulation Resistance, (6) DC Insulation Resistance, and (7) Line Leakage Current to name a few.

### STORAGE

In the event that a device or system of the invention has not been used for an extended period of time it should be prepared and stored in an appropriate manner. This includes at a minimum a) assure the control panel is key locked and that the key is not left accessible with the unit, b) coil the power cable and attached it to the unit in a manner such that it will not come free, drag on the floor or within the wheel area of the unit, c) make sure the cable and connector can not be bent or crushed in placement of unit for storage, d) cover the device or system completely in a manner to prevent dust or water from reasonably coming in to contact with the unit, and e) store in a temperature-controlled environment or in an area in which the temperature remains in the range of 4°C (40°F) to 49°C (1.20°F).

It is contemplated that a device of the present invention may be used under medical supervision for adjunctive therapy for the treatment of medical diseases and conditions. A device of the invention may be indicated for use in stimulating neuromuscular tissues for bulk muscle excitation in the legs or arms for rehabilitative purposes. Indications for use muscle stimulators include, but are not limited to relaxation of muscle spasms, increasing local blood circulation, muscle reeducation, prevention or retardation of disuse atrophy, immediate post-surgical stimulation of calf muscles to prevent venous thrombosis, or maintaining or increasing range of motion.

Magnetic therapy devices have been used for the relief of pain with demonstrated efficacy and safety. Other similar devices have delivered electromagnetic energy either transcutaneously or invasively for pain relief and to promote healing and bone growth.

### ELECTROMAGNETIC THERAPY

Electrical and magnetic nerve stimulation has been used for over a hundred years but more importantly over the past 20 years, with sound medical studies and supervision. The use has shown well-established positive results in pain relief without negative side effects.

Each stimulation device may have different body responses based on its particular delivery of electromagnetic energy. There exists an almost unlimited possible variation in the electromagnetic field, current, voltage, frequency, waveform and length of delivery of the energy.

The device of the present invention delivers electromagnetic energy at very low levels, well within parameters of other FDA approved magnetic stimulator devices. Its purpose and intended use is similar to other stimulator devices, to relieve pain. These devices are intended for use only for the relief of pain which has been determined by adequate medical diagnosis as being chronic or in response to conditions known and being treated.

The device of the present invention may be 2m (6½') long x 0.7m (2½') wide x 1.2m (4') high chamber. The device may have 1, 2, 3, 4, 5, 6, 7. 8, 9, 10 or more apertures ranging from 2, 2.5, 3, 35, 4 inches to 3, 3.5, 4, 4.5, 5, 5.5, 6, 6.5, 7, 7.5, 8, 8.5 or more inches in diameter. The apertures may be structured as "cuffs" through which an arm, leg or other body part may be inserted. Surrounding each cuff is a separately operated electromagnetic coil. A portion of a target body extremity is exposed to a continuous unipolar electromagnetic field modulated with a time-varying pulsating magnetic field component at a repetition rate of about 100, 110, 120 or 140 pulses per second. The total combined field may be several tenths of a Tesla in magnitude for a period of 10, 15, 20, 25, 30, 40, 50 to 60 minutes in duration. The body cell function, being complicated combinations of electrical and chemical ion interactions, may be directly affected by tube electromagnetic field.

Devices described herein provide delivery of electromagnetic energy in a form which provides many of the same type of cell interactions as other magnetic stimulation devices. The importance of this device is that it provides similar pain relief, but potentially affecting greater body volume and in some cases a longer lasting treatment effect, at significantly safer electromagnetic levels, with inherently a safer deliver device and more easily performed procedure.

### DIABETES

Diabetes is the most common chronic disease in the developed countries, with more than one hundred million cases diagnosed worldwide, and sixteen million diagnosed in the United States. It is the third most common cause of death in the U. S. Approximately one percent of all diabetes patients undergo amputations, a rate fifteen times higher than the non-diabetic population.

An estimated seventy percent of diabetic patients have nerve damage that impairs feeling in their feet. Fifteen percent of all diabetics eventually will develop a foot ulcer. Among those with ulcers, one in four will lose a foot. Every year, over 86,000 amputations are performed as a result of diabetes, and studies show that about one half of those who have a foot or leg amputated will lose the other one within five years.

Blindness and visual disability are caused due to diabetes mellitus. The blood vessels in the retina are damaged due to this chronic autoimmune disease which eventually results in loss of vision. It is possible to suggest from findings, consistent from study to study that approximately two percent of people become blind after fifteen years of diabetes, while severe visual handicaps are developed by about ten percent. Loss of vision from certain types of glaucoma and cataracts may be more common in people with diabetes than in those without it.

### The Electromagnetic Treatment Device (ETD)

The ETD is an experimental device that has been tested over the span of several years on thousands of human patients. There has been an on-going exchange of information regarding the ETD with the United States Food and Drug Administration (FDA). The FDA has ruled that it is a "non-significant-risk device." No adverse side effects have ever been noted in the patients treated, and the number of experimental treatments administered exceeds fifteen thousand. The ETD has been observed effective to a significant degree in reducing pain in a wide variety of diseases, injuries, and other medical conditions. Patients with various diabetic complications are among those whose pain scores have been shown reduced after treatments.

### Diabetes Maintenance

Type 1 diabetes requires that insulin be replaced directly. Type 2 diabetics have various drugs available to enhance the production of natural insulin rather than replacing it. Various combinations of drugs may be tried as the disease progresses. Eventually, natural insulin may completely fail and insulin replacement is needed.

Tight control of blood glucose levels delays the onset and slows progression of neuropathy. Certain drugs and even natural supplements have proved helpful. Tight insulin control is needed to help prevent retinopathy. Once damage to the eye develops, surgery may be needed.

Experimental treatment with the ETD has been demonstrated in previous studies to be an effective treatment modality for reducing the severity of symptoms of both diabetic neuropathy and diabetic retinopathy.

### SICKLE-CELL ANEMIA

Sickle cell disease is an inherited blood disorder characterized by defective .hemoglobin. It affects millions of people throughout the world and approximately 72,000 people in the U.S. It is present in one of every 500 African-American births.

Normal hemoglobin cells are smooth and round, allowing for ease in moving through blood vessels. Sickle cell hemoglobin molecules are stiff and form into the shape of a sickle or a scythe. They tend to cluster together, and cannot easily move through blood vessels. The cluster causes a blockage and stops the movement of oxygen-carrying blood. Sickle cells die after about 10 to 20 days, unlike normal hemoglobin cells, which live for up to 120 days. This results in a chronic short supply of red blood cells, which causes anemia. In this condition most or all of the normal hemoglobin (HbA) has been replaced with the sickle cell hemoglobin (HbS). This is referred to as HbSS. It is the most common and most severe form of the sickle cell variations. These persons suffer from a variety of complications due to the shape and thickness of the sickled cells. Severe and chronic anemia is also a common characteristic for children with HbSS. (www.umm.edu).

Current specific treatment regimens for those who suffer sickle cell anemia are determined by the treating physician according to the patient's age, overall health, and medical history. Also factors are the extent of the disease; tolerance for specific medications, procedures, or therapies; expectations for the course of the disease; and the patient's opinion or preference. Treatments may include pain medications (for sickle cell crises); drinking plenty of water daily (eight to ten glasses) or receiving fluid intravenously (to prevent and treat pain crises); blood transfusions to relieve anemia and to prevent stroke, and to dilute the HbS with normal hemoglobin to treat chronic pain, acute chest syndrome, splenic sequestration, and other emergencies; folic acid (to help prevent severe anemia); hydroxyurea, a medication that helps reduce the frequency of pain crises and acute chest syndrome, and may decrease the need for frequent blood transfusions; bone marrow transplant, which has been successful in curing some persons with sickle cell disease; and/or penicillin (to prevent infections).

The established efficacy of ETD therapy to either replace or augment any of the established treatment modalities would be a highly desirable event. Anecdotal evidence suggests the strong possibility of this event being accomplished.

### ACQUIRED IMMUNE DEFICIENCY SYNDROME (AIDS)

Acquired Immune Deficiency syndrome (AIDS) is an impairment of the body's ability to fight disease. It leaves the affected individual vulnerable to illnesses that a healthy immune system might overcome. AIDS patients are susceptible to diseases called opportunistic infections. These are illnesses due to organisms commonly found in the environment, and harmful only to an individual with a weakened immune system. This lack of an immune system alters in important ways, the lives of these patients. AIDS affects approximately two million people in the United States alone.

AIDS patients are affected by early symptoms that are usually benign and conspicuous but may include fatigue; loss of appetite; fever; night sweats; swollen glands (enlarged lymph nodes) in the neck, armpits or groin; unexplained weight loss, diarrhea, persistent cough, and various skin lesions. However, the symptoms may persist for months or worsen as opportunistic diseases exploit the body's collapsed defenses. Many (about 52%) develop an unusual pneumonia caused by the protozoan *Pneumucystis carinii.* Another third of patients exhibit a rare cancer of the skin, Kaposi's sarcoma (KS) or contract one of many opportunistic diseases caused by fungi (yeasts), viruses, bacteria, and protozoans.

AIDS may be divided into three groups: (i) AIDS patients having only Kaposi's sarcoma (KS) and no other symptom, (ii) AIDS patients having only severe opportunistic infections such as Pneumucystis carinii pneumonia (PCP), or Candida (thrush), (iii) a third group of patients has both KS and opportunistic infections. The third group suffers the most severe changes in their immune systems, and patients with KS alone the least severe.

The present treatment of AIDS includes the use of various pharmaceuticals; AL-721, Ampligen, Azidothymidine (AZT), Recombinant soluble CD4 (T4), Dextran sulfate, Dideoxycytidine (ddC), Foscarnet, Interferons, Ribaviron, among others, which are used alone or in combination. One goal of AIDS treatment is the complete suppression of the crisis without the development of collateral effects related to the medicine. Medicinal therapies are not sufficient to control the AIDS crisis. The recurring crisis have a lasting effect upon the cognitive and psychosocial development, as well as on the quality of life.

Recently, magnetic stimulation has been used with success for the treatment of AIDS. Although the mechanism of action remains unknown, the following hypothesis have been postulated: The first is the cancellation of AIDS symptoms is related to the destructive interference of a coherent magnetic wave, produced by stimulation, and changing the magnetic or electrical properties of the affected cells. A second hypothesis is that the magnetic stimulation produces an inhibition of the viral activity in the region that produces dysfunction of the immune system. A third hypothesis is that the magnetic fields alter the properties at the biologic membranes regulating a variety of ionic pumps.

Another hypothesis is based on studies that have shown that T4 cells directly or indirectly regulate immune function. Magnetic stimulation suppressed the activity of the endogenous opioid peptides. Some drugs, such as phenotoin, phenobarbital and sodium valproate reduce the concentration of beta-endorphins in rats, suggesting that the mechanism of action of these drugs may be partly behind the interaction with the endogenous opioid peptides.

In still further study, the application of magnetic fields has been shown to influence the activity of purkenji cells in the brain, and because it is known these cells participates heavily in the initiation, as well as the propagation of the cerebral activity, it is possible that the effects of magnetic fields in the reduction of AIDS symptoms may be related in part to its interference with the cerebral functions. Autopsies reveal signs of neurological disease m 80 to 90 percent of people who die from AIDS.

In yet another study the pineal gland is believed to be a magnetism-sensitive organ, it is conceivable that the effects of magnetic fields upon AIDS may be mediated via the pineal gland. It has been demonstrated that magnetic fields affect the pineal glands producing structural changes, reduction of the serotonin-N-acetyl tranferase and hydroxyindol-orthomethyl transferase; reduction of the nocturnal content of cyclic AMP; increase in glucose uptake; reduction in the production of melatonin; behavior opposite that which the rat exhibits with the administration of melatonin. All of these discoveries indicate that changes m the magnetic environment can influence the production of melatonin and the modulation of the circadian rhythms dependent on the pineal.

The following evidence supports the association between pineal gland activity and AIDS: a) melatonin influences the electric activity of the brain in human beings and animals; b) administration of tritiated melatonin shows an accumulation in the cerebral structures which integrate cerebral activity (i.e., the hippocampus and the cerebellum); c) the administration of melatonin produces dischronation of the electroencephalogram; d) the intraperitoneal administration of melatonin in small doses augments the discharge of neurons from the red nucleus; and e) a pinealectomy produces general paroxysmal crisis of slow waves with high amplitudes of cephalic center origin.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1

Patient was a woman suffering from sickle-cell anemia. The patient received three treatment using an ETD as described herein. Blood was drawn before and after the third treatment. Peripheral smears were evaluated for differential RBC counts.

**Pre-treatment** sickle-cell percentage was 25% (75% non-sickled cells). Post-treatment sickle-cell count was 10% (90% non-sickled cells). Inventors contemplate expanding these studies to greater number of patients to further support these results.

### EXAMPLE 2

One patient had epileptic attacks for 28 years, with an occurrence from 1 to 20 times per month, was selected for treatment by the apparatus described herein. After having been treated by this apparatus for about two months she substantially made better because at least two thirds of her attacks were relieved.

### EXAMPLE 3

Another patient, an epileptic woman who had experienced at least 8 attacks per month for 34 years, was also exposed to magnetotherapy and at the end of a six-week period she was evaluated. She had only four attacks which occurred during the first four weeks of the magnetotherapy period while the last three weeks of treatment no attacks occurred.

### EXAMPLE 4

A third patient, an epileptic woman who had suffered attacks for the last 9 years, was selected for magnetotherapy for a period of practically two months. Prior to her treatment, this patient had convulsive attacks with a frequency of 3 to 12 per month. Her attacks diminished up to 7 times during the two months evaluated, no attacks having occurred the last ten days of said period. The treatment was suspended because she became pregnant.

### EXAMPLE 5

A fourth patient, a 33-year-old epileptic woman, who has had from 1 to 3 attacks per month for 15 years, was treated for ten-weeks. The patient had only one attack at the beginning of the treatment, no attacks having occurred for the remaining period of magnetotherapy.

### EXAMPLE 6

A fifth patient, a 19-year-old woman who had from 8 to 20 epileptic attacks per month for 4 years, was treated by the apparatus and within the first 5 days this patient had 16 attacks. The following 10 days, the frequency of the attacks diminished to 4 attacks in this latter period and the last 10 days being evaluated she had only 3 attacks. A significant reduction in the occurrence of the epileptic attacks was noted.

The patients described in examples 2-6 have been treated to control their convulsive attacks by means of anticonvulsive medicine, such as Phenobarbital, phenytoin, valproic acid, primidone, carbamazepine, etc. Except for the third patient, all of the patients remain under treatment by the apparatus described herein and encouraging results have been obtained for each patient.

### EXAMPLE 7

The effectiveness of pain relief using the compositions and methods of the present invention has been documented in patients undergoing treatment using the inventive device and methods. An open label study of 79 subjects was made. The secondary effect of pain relief was observed and documented in patients being treated. The results of a study documenting pain relief are presented in Provisional Application, incorporated by reference in its entirety. The effect of ETD was measured for pain relief for a wide range of maladies causing pain. Included in the study were subjects suffering from poor circulation, arthritis, fibromyalgia, multiple sclerosis, back pain, neuropathy, diabetes, and other diseases. For 1,195 painful locations and compared before treatment and after treatment, there was an average reduction in pain level after treatment of 2.39 points, on a scale of 0 being no pain and 10 being unbearable pain.

### EXAMPLE 8

A contemplated treatment schedule for the treatment of AIDS comprises a) A double blind study, b) Patients will be selected on the basis of the complete break-down of their immune system. Only patients in severe crisis will be used. c) Patients will be numbered in consecutive integers. Patients with an odd number will be treated with a device described herein in the on position (relative to the generation of a magnetic field, the cooling system will be on continuously). Patients with an even number will be treated with such a device in the off position.

Patient will receive CBC-Diff, SMA-20, and immune deficiency panel blood test before the initial treatment. Patient will receive a one hour treatment. Patient will, immediately after treatment, be given another CBC-Diff, SMA 20, and immune deficiency panel blood test. This will be followed by three to five days while the results of the first treatment are tested. The Patient will receive another CBC-Diff; SMA 20, and immune deficiency panel blood test. Patient will receive the Second Treatment, it will be one hour duration. Immediately after the second treatment patient will receive another CBC-Diff, SMA 20, and immune deficiency panel blood test. This will be followed by three to five days, while the results are analyzed. Patient will receive another CBC-Diff SMA 20, and immune deficiency panel blood test. Patient Will receive a third treatment, it will be one hour in duration. Patient will receive another CBC-Diff, SMA 20, and immune deficiency panel blood test. After the results of this test are received, the Patient will receive another CBC-Diff, SMA 20, and immune deficiency panel blood test. The results of these tests, and charts are provide in Provisional Application, incorporated by reference in its entirety.

### EXAMPLE 9

Previous studies of a randomized, controlled double-masked design have shown that the ETD treatment for complications due to diabetes is effective. A further study is contemplated to confirm these findings, and to gain further data on efficacy of treatments. The study should be conducted in a location where a population exists that are inflicted with the disease at a rate higher than that found in some areas of the United States. A principal investigator (PI) will be located in such a location, and the PI will be experienced with extensive treatment of the disease. In addition to measuring subjective severity of symptoms, a blood test to show possible cell separation should be employed. A randomized sham treatment controlled double-masked study design will be used.

The study will be designed to assess clinical symptoms response and blood properties response after ETD treatment in patients who have complications due to diabetes mellitus, and to evaluate the safety of the ETD as a treatment modality.

The primary objectives include: 1) Determination of the extent of immediate pain relief as a result of treatment; 2) Determination of the progression of pain relief from one interval in the treatment process to the next interval, 3) Determination of the changes in blood properties as a result of treatment; 4) Determination of the progression of changes in the blood properties from one interval in the treatment process to the next interval; 5) Determination of the persistent effect of pain relief two weeks after treatment; 6) Determination of the persistent changes in the blood properties two weeks after treatment; 7) Determination of the extent of any visual acuity changes immediately after treatment; 8) Determination the progression of any visual acuity changes from one interval in the treatment process to the next interval; 9) Determination of the extent of any persistent changes in visual acuity two weeks after treatment; 10) Determination of the extent of immediate relief of numbness or tingling as a result of treatment; 11) Determination of the progression of relief from numbness or tingling from one interval in the treatment process to the next interval; and 12) Determination of the persistent effect of relief from numbness or tingling two weeks after treatment.

Secondary Objectives include: 1) Determination of deterioration of comfort levels regarding pain or other symptoms pertinent to diabetes complications diagnoses are noted after treatment, if any; and 2) Note any side effects, adverse or otherwise, that may be attributable to ETD treatment.

This is designed to be a Phase II trial to evaluate safety and to assess the treatment effect on clinical symptoms and blood properties of patients with complications due to diabetes. One hundred patients will comprise the study population. A lesser number may ultimately used, according to preliminary results experienced. A successful test result will be a net improvement effect for actively treated patients, less the net improvement effect for sham treated patients, of twenty per cent (20 %) of the patients treated. If 20 % of treated patients show improvement in numerical rating scale measurements, or in blood properties, the treatment will be deemed successful.

If preliminary results show that the net treatment effect is that forty per cent (40 %) or more of patients improve, either by numeric rating scale or by blood property changes, and if at least fifteen active-treatment patients have been treated, then the PI at his option, and with the written consent of the sponsor and the RB, may declare the study complete. Or, alternatively, at the PI's option, with written consent of the sponsor and the RB, the study may continue but the requirement for randomized, sham treatment, controlled double-masked methodology will be eliminated. In the event that this study continues on an open-label basis, any number less than one hundred patients that the PI, with the consent of the sponsor and the IRB, believes to be adequate for achieving of the primary and secondary objectives, will be the agreed number to complete the requirements of this study protocol. If the PI determines that the results are good enough to warrant discontinuing randomized sham double-masked treatments, then he should honor his therapeutic obligation by continuing the study to its logical conclusion by treating patients on an open label basis.

This research will follow a randomized sham-treatment controlled double-masked design. A randomization assistant, who will be a disinterested third party, and the machine operator will know the treatment control status, but subjects and other study personnel will not. The device has similar noise levels and temperature whether in active or sham treatment mode, and controls for mode selection will not be visible to masked personnel.

All accepted study patients, whether active treated or sham treated, will follow the following procedure. 1) Describe the intensity of pain, attributable to complications due to diabetes, experienced just prior to treatment with the ETD. Locations of pain and related intensity levels will be narrowly defined, such as which fingers or toes are in pain. The NIH numeric rating scale shall be used to define pain levels; 2) Determine the intensity of numbness or tingling, attributable to complications due to diabetes, experienced just prior to treatment with the ETD. Location of numbness or tingling and related intensity levels will be narrowly defined, such as which fingers or toes are experiencing the numbness or tingling. The NIH numeric pain scale will be used; 3) Read an eye chart to the smallest line possible prior to ETD treatment. A "Bailey Lovie Chart #4, National Vision Institute Of Australia, copyright 1978" or equivalent will be used; 4) Have blood drawn sufficient for a CBC test, and a peripheral smear prior to ETD treatment. Each smear will be photographed in sufficient magnification to reveal shape and clustering propensities of the blood cells; 5) After fifteen minutes of treatment by the ETD, repeat all the measurements and blood sampling described in steps 1 through 4 above; 6) After thirty minutes of treatment by the ETD, repeat all the measurements and blood sampling described in steps 1 through 4 above; 7) Two weeks after treatment by the ETD, the patient will come back to the treatment site. He or she will repeat all the measurements and blood sampling described in steps 1 through 4 above.

At the follow-up visit two weeks after treatment by the ETD, the patient will be asked to submit an opinion as to whether he or she received an active or sham treatment. After the measurements and blood sampling is completed according to steps outlined above, the subject patient will be informed as to whether he or she was an active or sham-treated patient. For those active-treated patients, their participation in the study will terminate at that time. For those sham-treated subject patients, an offer will be tendered, and encouraged, to participate further with an open label treatment with the ETD. If the patient agrees to further treatment, the information measured and blood sampling described above will be followed. Their participation in the study will then be concluded.

Each patient will be given a form after their original treatment, for them to list any antibiotics or blood transfusions that they may have received during the two weeks interval between ETD treatment and follow-up visit. The PI will use that information as potential confounding factors in his assessing the efficacy of ETD treatments.

The study population is male or female patients, ages 18 - 80, who meet eligibility requirements. Patients from all races or ethnic groups are invited to participate.

The following conditions must be met before a patient may be enrolled in this study. First, patients must be 18-80 years old. Ages below 18 or above 80 are acceptable with specific approval of PI. Second, medical records adequate to document that patient has Type 1 or Type 2 diabetes, with acute pain, chronic pain, or mixed pain, or acute numbness, or chronic numbness. Third, if patients are experiencing no pain or numbness at time of treatment, objective blood test information, the Fasting Plasmas. Glucose Test at the minimum, must be in file to reasonably document and predict presence of diabetes. Finally, written informed consent conforming to IRB guidelines must be given.

Any of the following conditions eliminates a patient from participating in this protocol. 1) Inadequate records to establish proof of having been diagnosed with diabetes; 2) Pregnant or breast-feeding; 3) Blood transfusion within thirty days prior to ETD treatment; 4) Received antibiotic treatment within 14 days prior to ETD treatment; 5) Contraindication to MRI; 6) Prior experience with ETD; or 7) Unwillingness or inability to conform to all the steps required to complete the study.

At the time the informed consent is obtained, qualified patients will be assigned a study number.

Patient evaluations will be directed toward the acquisition of data addressing the primary and secondary objectives of the study.

Primary Objectives - The numeric pain rating scores of each patient will be analyzed to measure the immediate effect of ETD treatment. Both pain and numbness criteria will be considered. The differential between the scores prior to treatment, after fifteen minutes of treatment, and after thirty minutes of treatment will be computed. A determination of whether the more significant change, if any, occurs in the first fifteen minutes or the second fifteen minutes of treatment is of great interest. Then the persistent effect of the pain and numbness scores will be analyzed after two weeks from treatment date, as compared to scores taken immediately prior to ETD treatment, to better evaluate the efficacy of the ETD treatment. The changes will finally be expressed as a mean change in the scores for the actual treatment cohort and the sham-treatment-control cohort, and compare the differences between the two. Each participant's perception of his or her being actually treated or sham treated will be compared with the results measured. A reliable indicator, even based as it is on subjective information, of the ETD's ability to mitigate pain and numbness will be established.

The CBC test and peripheral smear will provide more objective data to determine treatment efficacy. It is contemplated that clustered cells, if any, will separate from each other to some extent even after the first fifteen minutes of treatment. The peripheral smear, with ensuing photographs of it, will test and document this affect. Similarly, it is anticipated that after the full thirty minutes of treatment, further separation will occur. Correlation to subjective numerical pain and numbness ratings will be made. It is expected that at the two-week follow-up that the smears will show possibly more cell separation.

An attempt will be made to correlate changes in CBC indices results with changes in numerical pain and numbness ratings.

Secondary Objectives - All side effects will be noted and their relationships to the treatment process will be examined.

Safety Considerations - Magnetic field exposure from the ETD is much less than that with MRI. No precautions are required to shield patients or staff from magnetic field exposure, as the measured field strength dissipates to almost zero within approximately six inches from treatment portals. Despite the fact of considerable less ETD magnetic field strength than MRI, any patient contraindicated for MRI will not be allowed for treatment.

Adverse Experiences - All adverse experiences (AE) must be recorded and reported. The PI must also notify the RB. The sponsor will maintain records, and will include accounts of AE's, if pertinent, in future filings with the FDA.

Institutional Review - Prior to implementation of this study, the research protocol and the proposed patient consent form must be reviewed by a properly constituted Institutional Review Board. A signed and dated statement that they have approved the protocol must be submitted to the sponsor prior to the start of the study. This IRB must also approve all amendments to the protocol.

Informed Consent - Written consent will be obtained from each patient prior to entering the trial and will become part of the patient's permanent study record. Each patient will be assured that study participation is voluntary and that he/she may withdraw at any time, without penalty.

At the time of obtaining written consent, the investigator will advise patients of the experimental nature of the device, the duration of the trial, alternate modes of treatment, and prevalent adverse reactions that might occur. The patient's signature is to be witnessed.

Reporting and Recording of Data - All information required by the protocol is to be provided, or an explanation given for omissions. All data and information will be typed or legibly printed in black ink for ease of duplication interpretation and analysis.

Recruiting for this trial will be from the PI's clinical practice, from his professional colleagues, from word of mouth, and from newspaper, television, and interest-group advertising.

### EXAMPLE 10

Given the high degree of probability of pain relief in sickle cell anemia patients, based on antidotal evidence, and the absence of any probable adverse side effects as also determined by broad experience, a study is contemplated to test the ETD as a treatment modality for sickle cell anemia. The study should be conducted in a venue where there is a population largely at risk to the sickle cell trait. A principal investigator (PI) will be engaged, by the sponsor, that has a background in blood disorders and that has an abiding interest in breaking new ground in the treatment of the disease. The test should be somewhat unambiguous as to its results, so that reasonable conclusions may be drawn from the results. Subjective data (pain measurements) as well as objective data (blood tests) will be gathered, so a randomized sham-treatment controlled double-masked study design will be used to minimize any placebo effect.

This is a study designed to assess clinical symptoms response and blood properties response after ETD treatment in patients who have sickle cell anemia, and to evaluate the safety of the ETD as a treatment modality.

Primary Objectives are to determine the extent of immediate pain relief as a result of treatment, determine the progression of pain relief from one interval in the treatment process to the next interval, determine the changes in blood properties as a result of treatment, determine the progression of changes in the blood properties from one interval in the treatment process to the next interval; determine the persistent effect of pain relief two weeks after treatment; determine the persistent changes in the blood properties two weeks after treatment, determine the extent of any visual acuity changes immediately after treatment, determine the progression of any visual acuity changes from one interval in the treatment process to the next interval, determine the extent of any persistent changes in visual acuity two weeks after treatment, determine the extent of immediate relief of numbness or tingling as a result of treatment, determine the progression of relief from numbness or tingling from one interval in the treatment process to the next interval, determine the persistent effect of relief from numbness or tingling two weeks after treatment.

Secondary objectives are to determine if any deterioration of comfort levels regarding pain or other symptoms pertinent to sickle cell anemia diagnoses are noted after treatment and to note any side effects, adverse or otherwise, that may be attributable to ETD treatment.

This study design is a Phase II trial designed to evaluate safety and to assess the treatment effect on clinical symptoms and blood properties of patients with sickle cell anemia.

One hundred patients will comprise the study population. A lesser number may ultimately used, according to preliminary results experienced. A successful test result will be a net improvement effect for actively treated patients, less the net improvement effect for sham treated patients, of twenty per cent (20 %) of the patients treated. If 20 % of treated patients show improvement in numerical rating scale measurements, or in blood properties, the treatment will be deemed successful. If preliminary results show that the net treatment effect is that forty per cent (40 %) or more of patients improve, either by numeric rating scale or by blood property changes, and if at least fifteen active-treatment patients have been treated, then the PI at his option, and with the written consent of the sponsor and the IRB, may declare the study complete. Or, alternatively, at the PI'S option, with written consent of the sponsor and the IRB, the study may continue but the requirement for randomized, sham treatment, controlled double-masked methodology will be eliminated. In the event that this study continues on an open-label basis, any number less than one hundred patients that the PI, with the consent of the sponsor and the IRB, believes to be adequate for achieving of the primary and secondary objectives, will be the agreed number to complete the requirements of this study protocol. If the PI determines that the results are good enough to warrant discontinuing randomized sham double-masked treatments, then he should honor his therapeutic obligation by continuing the study to its logical conclusion by treating patients on an open label basis.

This study will follow a randomized sham-treatment controlled double-masked design. A randomization assistant, who will be a disinterested third party, and the machine operator will know the treatment control status, but subjects and other study personnel will not. The device has similar noise levels and temperature whether in active or sham treatment mode, and controls for mode selection will not be visible to masked personnel.

All accepted study patients, whether active treated or sham treated, will follow the following procedure. 1) Describe the intensity of pain, attributable to sickle cell anemia, experienced just prior to treatment with the ETD. Locations of pain and related intensity levels will be narrowly defined, such as which fingers or toes are in pain. The NIH numeric rating scale shall be used to define pain levels. 2) Determine the intensity of numbness or tingling, attributable to sickle cell anemia, experienced just prior to treatment with the ETD. Location of numbness or tingling and related intensity levels will be narrowly defined, such as which fingers or toes are experiencing the numbness or tingling. The NIH numeric pain scale will be used. 3) Read an eye chart to the smallest line possible prior to ETD treatment. A "Bailey Lovie Chart #4, National Vision Institute Of Australia, copyright 1978" or equivalent will be used. 4) Have blood drawn sufficient for a CBC test, and a peripheral smear, prior to ETD treatment. Each smear will be photographed in sufficient magnification to reveal shape and clustering propensities of the sickle cell hemoglobin molecules. 5) After fifteen minutes of treatment by the ETD, repeat all the measurements and blood sampling described in steps above. 6) After thirty minutes of treatment by the ETD, repeat all the measurements and blood sampling described above. 7) Two weeks after treatment by the ETD, the patient will come back to the treatment site. He or she will repeat all the measurements and blood sampling described above.

At the follow-up visit two weeks after treatment by the ETD, the patient will be asked to submit an opinion as to whether he or she received an active or sham treatment. After the measurements and blood sampling is completed according to steps above, the subject patient will be informed as to whether he or she was an active or sham-treated patient. For those active-treated patients, their participation in the study will terminate at that time. For those sham-treated subject patients, an offer will be tendered, and encouraged, to participate further with an open label treatment with the ETD. If the patient agrees to further treatment, the information measured and blood sampling described in steps above will be repeated. Their participation in the study will then be concluded.

Each patient will be given a form after their original treatment, for them to list any antibiotics or blood transfusions that they may have received during the two weeks interval between ETD treatment and follow-up visit. The PI will use that information as potential confounding factors in his assessing the efficacy of ETD treatments.

The study population is male or female patients, ages 18 - 80 years, who meet eligibility requirements. Patients from all races or ethnic groups are invited to participate.

Inclusion Criteria - The following conditions must be met before a patient may be enrolled in this study. Patients age is 18 - 80 years. Ages below 18 or above 80 are acceptable with specific approval of PI. Medical records adequate to document that patient has sickle cell anemia, with acute pain, chronic pain, or mixed pain. If patients are experiencing no pain or numbness at time of treatment, objective blood test information must be in file to reasonably document and predict presence of sickled cells in patients' blood. Written informed consent conforming to IRB guidelines.

Exclusion Criteria - Any of the following conditions eliminates a patient from participating in this protocol: 1) Pregnant or breast-feeding; 2) Contraindication to MRI; 3) Prior experience with ETD; 4) Inadequate records to establish proof of having been diagnosed with sickle cell anemia; 5) Blood transfusion within thirty days prior to ETD treatment; 6) Received antibiotic treatment within 14 days prior to ETD treatment; or 7) Unwillingness or inability to conform to all the steps required to complete the study.

At the time the informed consent is obtained, qualified patients will be assigned a study number.

Patient evaluations will be directed toward the acquisition of data addressing the primary and secondary objectives of the study.

Primary Objectives - The numeric pain rating scores of each patient will be analyzed to measure the immediate effect of ETD treatment. Both pain and numbness criteria will be considered. The differential between the scores prior to treatment, after fifteen minutes of treatment, and after thirty minutes of treatment will be computed. A determination of whether the more significant change, if any, occurs in the first fifteen minutes or the second fifteen minutes of treatment is of great interest. Then the persistent effect of the pain and numbness scores will be analyzed after two weeks from treatment date, as compared to scores taken immediately prior to ETD treatment, to better evaluate the efficacy of the ETD treatment. The changes will finally be expressed as a mean change in the scores for the actual treatment cohort and the sham-treatment-control cohort, and compare the differences between the two. Each participant's perception of his or her being actually treated or sham treated will be compared with the results measured. A reliable indicator, even based as it is on subjective information, of the ETD's ability to mitigate pain and numbness will be established.

The CBC test and peripheral smear will provide more objective data to determine treatment efficacy. It is contemplated that the clustered sickled cells will separate from each other to some extent even after the first fifteen minutes of treatment. The peripheral smear, with ensuing photographs of it, will test and document that expectation. Similarly, it is anticipated that after the full thirty minutes of treatment, further separation will occur. Correlation to subjective numerical pain and numbness ratings will be made. It is expected that at the two-week follow-up that the smears will show possibly more cell separation.

Particular attention will be given to results of the CBC test in three areas, especially in the comparison between the prior-to-treatment CBC and the two-week follow-up CBC. These areas of interest are as follows. 1) Red blood cell (RBC) count. This index is a measure of the ability of the blood to carry oxygen from the lungs to the rest of the body. A low level of RBC is common to sickle cell anemia. A significant increase after ETD treatment would be a positive event. 2) Hematocrit. This index measures the amount of space or volume red blood cells occupy in the blood. An increase in hematocrit after ETD treatment would be a positive event. 3) Red cell distribution width (RDW). The RDW reports whether all the red cells are about the same width, size, and shape. This information will be helpful in assessing the level of anemia, and an improvement in the RDW over the two-week follow-up period would be a positive event.

An attempt will be made to correlate changes in CBC indices with changes in numerical pain and numbness ratings.

Secondary Objectives - All side effects will be noted and their relationships to the treatment process will be examined.

Safety Considerations - Magnetic field exposure from the ETD is much less than that with MRI. No precautions are required to shield patients or staff from magnetic field exposure, as the measured field strength dissipates to almost zero within approximately six inches from treatment portals. Despite the fact of considerable less ETD magnetic field strength than MRI, any patient contraindicated for MRI will not be allowed for treatment.

Adverse Experiences - All adverse experiences (AE) must be recorded and reported. The PI must also notify the RB. The sponsor will maintain records, and will include accounts of AE's, if pertinent, in future filings with the FDA.

Institutional Review - Prior to implementation of this study, the research protocol and the proposed patient consent form must be reviewed by a properly constituted Institutional Review Board. A signed and dated statement that they have approved the protocol must be submitted to the sponsor prior to the start of the study. This IRB must also approve all amendments to the protocol.

Informed Consent - Written consent will be obtained from each patient prior to entering the trial and will become part of the patient's permanent study record. Each patient will be assured that study participation is voluntary and that he/she may withdraw at any time, without penalty.

At the time of obtaining written consent, the investigator will advise patients of the experimental nature of the device, the duration of the trial, alternate modes of treatment, and prevalent adverse reactions that might occur. The patient's signature is to be witnessed.

Reporting and Recording of Data - All information required by the protocol is to be provided, or an explanation given for omissions. All data and information will be typed or legibly printed in black ink for ease of duplication interpretation and analysis.

Recruiting for this trial will be from the PI's clinical practice, from his professional colleagues, from word of mouth, and from newspaper, television, and interest-group advertising.

## Claims

1. A system for treating a human being comprising:
an electrical current source (10);
a housing (18) with an aperture (48) that permits insertion of a human appendage wherein said aperture is lined with a tubular inner sleeve (37);
a coil (14) that generates a magnetic field coupled to said electrical current source wherein said coil surrounds said aperture (48) and generates a magnetic field within said aperture; and
an insulating sleeve (38) located within the coil (14) wherein the aperture (48) and the tubular inner sleeve (37) are located within the insulating sleeve (38);
wherein said coil (14) is configured such that an appendage placed within said aperture is exposed to a magnetic field of between 100 gauss and 700 gauss and **characterized in that** the system comprises a gap provides between the insulating sleeve (38) and the tubular inner sleeve (37) to allow a cooling liquid to flow between.

2. The system of Claim 1 wherein said electrical current source is a direct current electrical current source.

3. The system of Claim 1 or Claim 2 wherein said electrical current source produces a modulated current flow.

4. The system of Claim 3 wherein said modulated current source has at least 100 pulses per second.

5. The system of any of the preceding claims wherein said tubular inner sleeve is comprised of a first type of metal and said housing is comprised of a second type of metal wherein the extraneous magnetic field outside of said housing is less than the extraneous magnetic field outside of said housing of a system where said housing is comprised of said first type of metal.

6. The system of any of the preceding claims wherein said housing is comprised of ferrous metal and said tubular inner sleeve is comprised of stainless steel.

7. The system of any of the preceding claims wherein the diameter of said coil is greater than the depth of said coil.

8. The system of any of the preceding claims further comprising a means for cooling said coil.

9. The system of any of the preceding claims further comprising a cooling liquid configured to absorb heat generated by said coil wherein the cooling liquid is pumped through an inlet on one surface of the housing and out an outlet on another surface of the housing.

10. The system of any of the preceding claims wherein the strength of said magnetic field within said aperture is substantially homogenous.

11. The system of any of the preceding claims wherein said magnetic field is a direct current magnetic field.

12. The system of any of the preceding claims wherein said magnetic field is a continuous unipolar magnetic field modulated with a time-varying pulsating magnetic field component.

13. The system of Claim 12 wherein said time-varying pulsating magnetic field component has a rate of at least 100 pulses per second, and preferably no greater than 140 pulses per second.

14. The system of any of the preceding claims further comprising a coil spindle plate mounted with the coil within the housing, the coil spindle plate having perforations allowing fluid to pass through the coil spindle plate to the coil.

## Patentansprüche

1. Ein System zum Behandeln eines Menschen, umfassend:
eine elektrische Stromquelle (10);
ein Gehäuse (18) mit einer Öffnung (48), die das Einrühren einer menschlichen Gliedmaße gestattet, während die Öffnung mit einer rohrförmigen inneren Hülse (37) ausgekleidet ist;
eine Spule (14), die ein magnetisches Feld erzeugt, das an die elektrische Stromquelle gekoppelt ist, während die Spule die Öffnung (48) umgibt und ein magnetisches Feld innerhalb der Öffnung erzeugt; und
eine Isolierhülse (38), die innerhalb der Spule (14) angeordnet ist, während die Öffnung (48) und die rohrförmige innere Hülse (37) innerhalb der Isolierhülse (38) angeordnet sind,
während die Spule (14) konfiguriert ist, sodass eine Gliedmaße, die innerhalb der Öffnung angeordnet wird, einem magnetischen Feld zwischen 100 Gauß und 700 Gauß ausgesetzt ist, **dadurch gekennzeichnet, dass** das System einen Spalt zwischen der Isolierhülse (38) und der rohrförmigen inneren Hülse (37) bereitstellt, um es zu erlauben, dass eine Kühlflussigkeit dazwischen fließt.

2. Das System gemäß Anspruch 1, in dem die elektrische Stromquelle eine elektrische Gleichstromquelle ist.

3. Das System gemäß Anspruch 1 oder Anspruch 2, in dem die elektrische Stromquelle einen modulierten Stromfluss erzeugt.

4. Das System gemäß Anspruch 3, in dem die modulierte Stromquelle mindestens 100 Impulse pro Sekunde aufweist.

5. Das System gemäß einem der vorhergehenden Ansprüche, in dem die rohrförmige innere Hülse aus einer ersten Metallart und das Gehäuse aus einer zweiten Metallart besteht, wobei das äußere magnetische Feld außerhalb des Gehäuses kleiner ist als das äußere magnetische Feld außerhalb des Gehäuses eines Systems, in dem das Gehäuse aus dem ersten Metalltyp besteht.

6. Das System gemäß einem der vorhergehenden Ansprüche, in dem das Gehäuse aus einem Eisenmetall besteht und die röhrförmige innere Hülse aus einemlrostfreien Stahl.

7. Das System gemäß einem der vorhergehenden Ansprüche, in dem der Durchmesser der Spule größer ist als die Tiefe der Spule.

8. Das System gemäß einem der vorhergehenden Ansprüche, umfassend ein Mittel zum Kühlen der Spule.

9. Das System gemäß einem der vorhergehenden Ansprüche, weiterhin umfassend, eine Kühlflüssigkeit, die konfiguriert ist, um Wärme zu absorbieren, die durch die Spule erzeugt wurde, während die Kühlflüssigkeit durch einen Einlass auf einer Oberfläche des Gehäuses und aus einem Auslass auf einer weiteren Oberfläche des Gehäuses gepumpt wird.

10. Das System gemäß einem der vorhergehenden Ansprüche, in dem die Stärke des magnetischen Felds innerhalb der Öffnung im Wesentlichen homogen ist.

11. Das System gemäß einem der vorhergehenden Ansprüche, in dem das magnetische Feld ein magnetisches Gleichstromfeld ist.

12. Das System gemäß einem der vorhergehenden Ansprüche, in dem das magnetische Feld ein kontinuierliches unipolares magnetisches Feld ist, das mit einer zeitlich variierenden pulsierenden magnetischen Feldkomponente moduliert wird.

13. Das System gemäß Anspruch 12, in dem die zeitlich variierende pulsierende magnetische Feldkomponente eine Rate von mindestens 100 Impulsen pro Sekunde und vorzugsweise nicht mehr als 140 Impulse pro Sekunde aufweist.

14. Das System gemäß einem der vorhergehenden Ansprüche, umfassend eine Spulenspindelplatte, die mit der Spule innerhalb des Gehäuses befestigt ist, während die Spulenspindelplatte Perforationen aufweist, die es erlauben, dass Flüssigkeit durch die Spulenspindelplatte zu der Spule fließt.

## Revendications

1. Système de traitement d'un être humain comprenant:
une source de courant électrique (10);
un boîtier (18) avec une ouverture (48) qui permet l'insertion d'un appendice humain lorsque ladite ouverture est garnie d'un manchon interne tubulaire (37);
une spire (14) qui produit un champ magnétique couplé à ladite source de courant électrique, où ladite spire entoure ladite ouverture (48) et produit un champ magnétique dans ladite ouverture; et
un manchon d'isolation (38) situé dans la spire (14), l'ouverture (48) et le manchon interne tubulaire (37) étant situés dans le manchon isolant (38);
où ladite spire (14) est configurée de façon qu'un appendice placé dans ladite ouverture soit exposé à un champ magnétique compris entre 100 Gauss et 700 Gauss, et **caractérisé en ce que** le système comprend un espace réalisé entre le manchon isolant (38) et le manchon interne tubulaire (37) pour permettre à un liquide de refroidissement de s'écouler entre eux.

2. Système selon la revendication 1, dans lequel ladite source de courant électrique est une source de courant électrique de courant direct.

3. Système selon la revendication 1 ou la revendication 2, dans lequel ladite source de courant électrique produit un écoulement de courant modulé.

4. Système selon la revendication 3, dans lequel ladite source de courant modulé a au moins 100 impulsions par secondes.

5. Système selon l'une quelconque des revendications précédentes, dans lequel le manchon tubulaire interne est constitué d'un premier type de métal, et ledit boîtier est constitué d'un deuxième type de métal, où le champ magnétique parasite à l'extérieur dudit boîtier est plus petit que le champ magnétique parasite à l'extérieur dudit boîtier d'un système où ledit boîtier est constitué dudit premier type de métal.

6. Système selon l'une quelconque des revendication précédentes, dans lequel ledit boîtier est constitué de métal ferreux, et ledit manchon tubulaire interne est constitué d'acier inoxydable.

7. Système selon l'une quelconque des revendications précédentes, dans lequel le diamètre de ladite spire est supérieur à la profondeur de ladite spire.

8. Système selon l'une quelconque des revendications précédentes, comprenant en outre un moyen de refroidissement de ladite spire.

9. Système selon l'une quelconque des revendications précédentes, comprenant en outre un liquide de refroidissement configuré pour absorber la chaleur produite par ladite spire lorsque le liquide de refroidissement est pompé à travers une entrée sur une surface du boîtier et hors d'une sortie sur une autre surface du boîtier.

10. Système selon l'une quelconque des revendications précédentes, dans lequel la force dudit champ magnétique dans ladite ouverture est sensiblement homogène.

11. Système selon l'une quelconque des revendications précédentes, dans lequel ledit champ magnétique est un champ magnétique à courant direct.

12. Système selon l'une quelconque des revendications précédentes, dans lequel ledit champ magnétique est un champ magnétique unipolaire continu modulé par un composant de champ magnétique à impulsions variées dans le temps.

13. Système selon la revendication 12, dans lequel ladite composante de champ magnétique à impulsions variées dans le temps a un débit d'au moins 100 impulsions par seconde, et de préférence pas plus que 140 impulsions par seconde.

14. Système selon l'une quelconque des revendications précédentes, comprenant en outre une plaque de broche de spire sur laquelle est montée la spire dans le boîtier, la plaque de broche de spire ayant des perforations permettant au fluide de passer à travers la plaque de broche de spire vers la spire.
